(19) Europäisches Patentamt
European Patent Office
Office européen des brevets

(11) Veröffentlichungsnummer : **0 303 562 B1**

(12) # EUROPÄISCHE PATENTSCHRIFT

(45) Veröffentlichungstag der Patentschrift : **15.01.92 Patentblatt 92/03**

(51) Int. Cl.[5] : **C07C 405/00**, C07F 7/18, C07C 69/73

(21) Anmeldenummer : **88730179.4**

(22) Anmeldetag : **11.08.88**

(54) **Verfahren zur Herstellung von E/Z-Gemischen von 2-(Bicyclo[3.3.0]octan-3-yliden)-essigsäurederivaten mit überwiegendem E- oder Z-Anteil.**

(30) Priorität : **11.08.87 DE 3727065**

(43) Veröffentlichungstag der Anmeldung : **15.02.89 Patentblatt 89/07**

(45) Bekanntmachung des Hinweises auf die Patenterteilung : **15.01.92 Patentblatt 92/03**

(84) Benannte Vertragsstaaten : **AT BE CH DE ES FR GB GR IT LI LU NL SE**

(56) Entgegenhaltungen :
**EP-A- 0 119 949**
**CHEMISTRY AND INDUSTRY, Nr. 50, 15. Dezember 1962, Seiten 2085-2086, London, GB; I. TÖMÖSKÖZI et al.: "Asymmetric induction in the wittig reaction"**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Rehwinkel, Hartmut, Dr.**
**Glasower Strasse 41**
**W-1000 Berlin 44 (DE)**
Erfinder : **Vorbrüggen, Helmut, Prof.**
**Wilkestrasse 7**
**W-1000 Berlin 27 (DE)**
Erfinder : **Gais, Hans-Joachim, Prof.**
**In den Weihermatten 28**
**W-7800 Freiburg (DE)**
Erfinder : **Schmiedl, Gerhard**
**Reichenbachstrasse 2**
**W-7800 Freiburg (DE)**
Erfinder : **Bund, Jörg**
**Schwarzwaldstrasse 19**
**W-7800 Freiburg (DE)**

**EP 0 303 562 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Optisch aktives 6α-Carbacyclin und besonders einige davon abgeleitete Verbindungen sind als stabile Analoga des natürlichen Prostacyclins ($PGI_2$) therapeutisch sehr interessant. [R.C. Nickolson, M.H. Town, and H. Vorbrüggen, Medicinal Research Reviews 5, 1 (1985)]

Bei der Synthese der 6α-Carbacycline entstehen, wenn man die obere Kette über eine Wittig-Reaktion einführt, immer ca. 50% der biologisch potenten 5E- und ca. 50% der biologisch fast inaktiven 5Z-Analoga.

Asymmetrische Horner-Emmons-Olefinierungen bzw. verwandte Reaktionen zur Erzeugung der Doppelbindung wurden bisher nur von Tömösközi und Janzso (Chem. Ind. 1962, 2085) beschrieben, die (–)-Methylphosphonoacetat-P,P-diethylester mit 4-substituierten Cyclohexanonen umsetzen. Die optischen Ausbeuten, die sie erhielten, waren jedoch sehr gering.

Ferner beschrieben Bestmann und Lienert [Angew. Chem. 81, 751 (1969)] eine Synthese von optisch aktiven Benzylidencyclohexanen aus 4-substituierten Cyclohexanonen und dem relativ schwer zugänglichen (R)-Benzyliden-methyl-phenyl-n-propylphosphoran.

Hanessian und Mitarbeiter setzten außerdem 3- und 4-substituierte Cyclohexanone mit chiralen Phosphonamiden zur Einführung der optischen Aktivität um [J. Amer. Chem. Soc. 106, 5754 (1984)].

Schließlich erhielten Erdelmeier und Gais aus 7,7-Ethylendioxy-bicyclo[3.3.0]octan-3-on und lithiierten chiralen Sulfoximinen unsymmetrische Olefine [Angew. Chem. 98, 912 (1986)].

Es wurde nun überraschend gefunden, daß bei der Umsetzung von Bicyclo[3.3.0]octan-3-on derivaten mit chiralen Phosphonaten die entsprechenden 2-(Bicyclo[3.3.0] octan-3-yliden)-essigsäurederivate mit unerwartet hohem E- bzw. Z-Anteil entstehen.

Die Erfindung betrifft somit ein Verfahren zur Herstellung von E/Z-Gemischen von 2-(Bicyclo[3.3.0]octan-3-yliden)-essigsäurederivaten der Formel I,

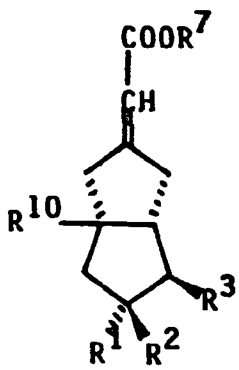

(I),

in denen entweder der E- oder der Z-Anteil überwiegt, worin

$R^1$ $OCH_3$, $OC_2H_5$,

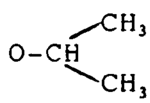

und, wenn $R^2$ Wasserstoff darstellt, den Rest $OR^4$ mit $R^4$ in der Bedeutung eines Wasserstoffs, Trialkylsilyls, Diphenyl-alkylsilyls, tert.-Butyl-methoxy-phenylsilyls, Trityls, Tetrahydropyranyls, $C_7$-$C_{11}$-Aroyls oder $C_1$-$C_6$-Acyls,

$R^2$ $OCH_3$, $OC_2H_5$,

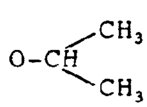

,

$R^1$ und $R^2$ gemeinsam die Reste

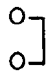, 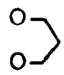, 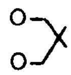 oder 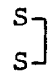,

| | |
|---|---|
| $R^3$ | Wasserstoff, $—CH_2OR^4$ mit $R^4$ in der oben angegebenen Bedeutung oder $—A\text{-}W\text{-}D\text{-}E\text{-}R^5$, worin |
| A | eine trans-CH=CH—, eine —CH=CBr— oder —C=C-Gruppe, |
| W | die Reste |

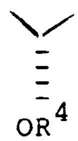 oder 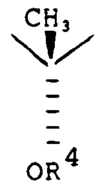

| | |
|---|---|
| | mit |
| $R^4$ | in der oben angegebenen Bedeutung, |
| D | eine geradkettige Alkylengruppe mit 1-7 C-Atomen oder eine verzweigkettige Alkylengruppe mit 2-7 C-Atomen, |
| E | eine —C≡C—, $—CH=CR^6—$, $—O\text{-}R^5$ oder $—R^5$-Gruppe, |
| $R^5$ | eine Alkylgruppe mit 1-6 C-Atomen, |
| $D\text{-}E\text{-}R^5$ | eine Cycloalkylgruppe mit 3-8 C-Atomen oder den Rest |

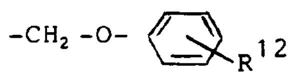,

| | |
|---|---|
| $R^6$ | eine Alkylgruppe mit 1-6 C-Atomen oder Halogen, |
| $R^{12}$ | Wasserstoff, 4-Halogen oder 3-Trifluormethyl sein können, |
| $R^7$ | (+)- oder (–)-Menthyl, (+)- oder (–)-8-Arylmenthyl, (+)-8-Arylneomenthyl, (+)- oder (–)-trans-2-Arylcycloalkyl mit 3-8 C-Atomen im cycloalkyl und mit Aryl als gegebenenfalls substituiertes Phenyl, 1-oder 2-Naphthyl oder 1-, 2- oder 9-Anthranyl, gegebenenfalls substituiertes Bornyl oder 3-Methoxy-1, 3,5-estratrien-17β-yl und |
| $R^{10}$ | Wasserstoff, Methyl, Ethinyl, 1-Propinyl oder den Rest $—C{\equiv}C\text{-}(CH_2)_m\text{-}R^{11}$, worin |
| m | 2-20 und |
| $R^{11}$ | Wasserstoff, Azido, Amino, Methylamino, Benzylamino, Carboxyl, Methoxycarbonyl, Ethoxycarbonyl, Benzyloxycarbonyl, Hydroxy, Cyano, Brom, Chlor oder Jod darstellen, |

bedeuten, das dadurch gekennzeichnet ist, daß man ein Bicyclo[3.3.0]octan-3-on der Formel II,

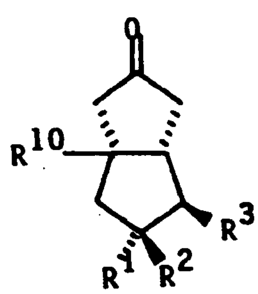 (II),

worin $R^1$, $R^2$, $R^3$ und $R^{10}$ die oben angegebenen Bedeutungen haben, mit einem chiralen Phosphonat der Formel III,

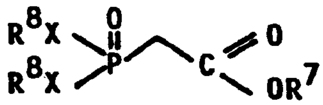

(III),

worin

X Sauerstoff oder den Rest —N($R^9$)—,
$R^8$ einen geradkettigen oder verzweigten Alkylrest mit 1-6 C-Atomen, Phenyl oder 2,2,2-Trifluorethyl oder beide Reste $R^8$ gemeinsam 1,2-Cyclohexyliden und
$R^9$ Wasserstoff, Methyl, Ethyl oder Benzyl bedeuten und
$R^7$ die oben angegebene Bedeutung hat,

in Gegenwart eines Deprotonierungsmittels umsetzt.

Wenn $R^4$ Trialkylsilyl oder Diphenyl-alkylsilyl bedeutet, so sind damit Gruppen mit $C_1$-$C_4$-Alkyl im alkyl-Teil gemeint, wie z.B. Trimethylsilyl, Dimethyl-tert.-butyl-silyl, Triethylsilyl, Diphenyl-tert.-butyl- bwz.-methylsilyl.

Die OH-Gruppe (für $OR^4$) kann durch die dem Fachmann bekannten Schutzgruppen geschützt werden.

$R^4$ als $C_1$-$C_6$-Acyl bedeutet Formyl, Acetyl, Propionyl, Butyryl, Pentanoyl, Hexanoyl. Bevorzugt sind die $C_2$-$C_4$-Acyle.

$R^4$ als $C_7$-$C_{11}$-Aroyl bedeutet Benzoyl, Naphthoyl, bevorzugt jedoch Benzoyl.

D als geradkettige Alkylengruppe mit 1-7 C-Atomen soll sein : methylen, ethylen, trimethylen, tetramethylen u.s.w.. Bevorzugte geradkettige Alkylengruppen D sind die mit 2-4 C-Atomen. Für die verzweigte Alkylengruppen D mit 2-7 C-Atomen gilt ähnliches. In Betracht kommen die Gruppen

$$-\underset{CH_3}{\underset{|}{CH}}-,\quad -\underset{CH_3}{\underset{|}{CH}}-CH_2-,\quad -C(CH_3)_2-CH_2-,\quad -\underset{CH_3}{\underset{|}{CH}}-CH_2-CH_2-,\quad -C(CH_3)_2-CH_2-CH_2-,$$

$$-\underset{CH_3}{\underset{|}{CH}}-(CH_2)_3-,\quad -C(CH_3)_2-(CH_2)_3-\ \text{u.s.w..}$$

Bevorzugt verzweigte Alkylengruppen D sind die mit 2-4 C-Atomen in der Kette.

$R^5$, $R^6$ und $R^8$ als Alkylgruppen mit 1-6 C-Atomen sollen sein Methyl, Ethyl, n-Propyl, Isopropyl, n-Butyl, sek.-Butyl, Isobutyl, tert.-Butyl, Pentyl, Hexyl. Bevorzugte Alkylgruppen $R^5$ und $R^6$ sind die mit 1-4 C-Atomen. Bevorzugte Alkylgruppen $R^8$ sind die mit 1-3 C-Atomen.

DE$R^5$ als Cycloalkylgruppe mit 3-8 C-Atomen bedeutet Cyclopropyl, Cyclobutyl, Cyclopentyl, Cyclohexyl, Cycloheptyl, Cyclooctyl. Bevorzugt sind die Gruppen mit 4-6 C-Atomen. Wenn $R^{12}$ Halogen bedeutet, so kommen Chlor und Brom in Frage. Unsubstituiertes Phenyl soll für Aryl (als Substituent in den Resten $R^7$) bevorzugt sein.

Die chiralen Phosphonate III (wenn X = 0) werden durch Aktivierung des Phosphonoessigsäure-P,P-dimethylesters (Coutrot et al., Synthesis 1978, 133) mit DCC/DMAP [Neises und Steglich, Angew. Chem. 90, 556 (1978)] bzw. mit Oxalylchlorid [Miyano und Dorn, J. Org. Chem. 37, 268 (1972)] und anschließender Umsetzung der aktivierten Stufe mit den entsprechenden optisch aktiven Alkoholen dargestellt. Auch durch Erhitzen der Säuren mit den chiralen Alkoholen in Gegenwart von p-Toluolsulfonsäure-Hydrat am Wasserabscheider entstehen die gewünschten Produkte III. DMAP-katalysierte Umesterung von Trialkylphosphonoacetat mit asymmetrischen Alkoholen wie Phenylmenthol führt ebenfalls zum Ziel [Hatekeyama et al., Tetrahedron Lett. 28, 2713 (1987)].

Die chiralen Phosphonate III mit X = N-$R^9$ erhält man durch Umsetzung von Methanphosphonsäuredichlorid mit den entsprechenden Aminen in Gegenwart von Pyridin und anschließender Alkylierung des entstandenen Produkts mit den entsprechenden Halogenessigsäureestern.

Nach dem erfindungsgemäßen Verfahren lassen sich selektiv entweder (E)- oder (Z)-2-(Bicyclo[3.3.0]octan-3-yliden)-essigsäurederivate in hohen Ausbeuten darstellen. So vereinfachen sich aufwendige chromatographische Trennungen von (1 : 1)-E/Z-Gemischen, die man nach den bisherigen Verfahren erhält.

Die selektiv hergestellten (E)-Ester der allgemeinen Formel IV ergeben bei der Reduktion, insbesondere mit DIBAH, die entsprechenden (E)-Allylalkohole V, die wichtige Zwischenprodukte für die Darstellung von chemisch und metabolisch stabilen 3-Oxacarbacyclinen wie z.B. von Cicaprost und Eptaprost sind [vgl. W. Skuballa et al., J. Med. Chem. 29, 313 (1986)].

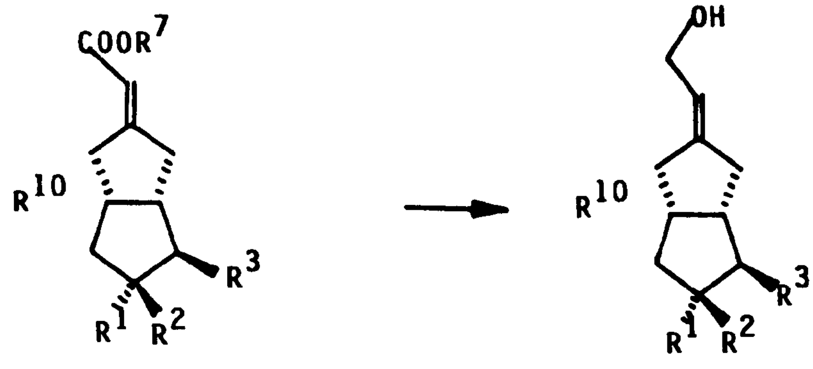

IV V

Diese beiden Wirkstoffe senken den Blutdruck und hemmen die Thrombozytenaggregation. Um ausgehend von V zu diesen biologisch wirksamen Verbindungen zu kommen, schließt sich an die Reduktion von IV zu V eine Veretherung an, wie sie z.B. in DE-OS 3306123 beschrieben wird [vgl. auch W. Skuballa et al., J. Med. Chem. 29, 313 (1986)].

Nach neueren synthetischen Arbeiten lassen sich die Derivate VI der Allylalkohole V, in denen X eine austretende Gruppe (X = Br, OTs, OMs, OAc) darstellt, mit metallorganischen Reagenzien wie z.B. Zn- bzw. Li-Organylen oder auch Grignard-Verbindungen in die entsprechenden Carbacycline VII überführen [vgl. E. Nakamura et al., Tetrahedron Lett. 28, 337 (1987) ; Y. Tanigawa et al., J. Am. Chem. Soc. 99, 2361 (1977) ; G. Fouquet und M. Schlosser, Angew. Chem. 86, 50 (1974].

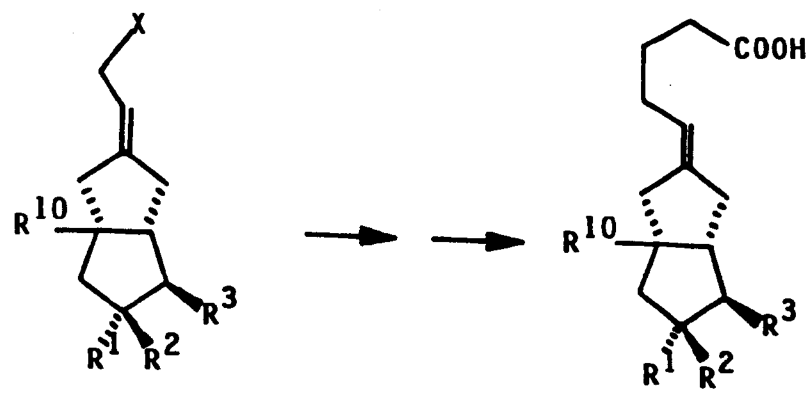

VI VII

Bei der Umsetzung von II mit III sind die molaren Verhältnisse der Reaktionspartner variierbar. Als vorteilhaft erweist es sich jedoch, einen Überschuß des chiralen Phosphonats III (bezogen auf das Keton) sowie einen Unterschuß an Deprotonierungsmittel (bezogen auf das Phosphonat III) einzusetzen, da durch diese Maßnahmen die Bildung unerwünschter Isocarbacyclinderivate (bei denen die exo-Doppelbindung in den 5-Ring gewandert ist) weitgehend unterdrückt wird.

Als Deprotonierungsmittel sind die dem Fachmann bekannten Basen wie Alkalihydride, Alkaliamide, Kalium-tert.-butylat, Alkalisalze des Hexamethyldisilazans, BuLi, LDA, KDA sowie Alkalisalze asymmetrischer Basen wie z.B. (R,R)- oder (S,S)-Bis-(1-phenylethyl)-amin [vgl. J.A. Marshall et al., Tetrahedron Lett. 28, 3323 (1987)] geeignet. Als besonders vorteilhaft für die Erzielung eines hohen E/Z-Verhältnisses erweisen sich kaliumhaltige Basen.

Die Umsetzung von II und III wird bei Temperaturen von – 70°C bis + 44°C, insbesondere bei – 40°C bis + 40°C durchgeführt.

Als Lösungsmittel für die Durchführung der Reaktion eignen sich übliche Lösungsmittel wie z.B. Tetrahydrofuran, Diethylether, Toluol, Gemische aus Tetrahydrofuran und Toluol, 1,2-Glykoldimethylether, Diethylenglykoldimethylether.

Zur Bestimmung des E/Z-Verhältnisses werden die E/Z-2(Bicyclo[3.3.0]octan-3-yliden)-essigsäureester entweder direkt gaschromatographisch untersucht oder nach Reduktion mit DIBAH und anschließender Umsetzung mit N-Methyl-N-(trimethylsilyl)-2,2,2-trifluoracetamid (MSTFA) ebenfalls gaschromatographisch bei Temperaturen zwischen 100 und 300°C getrennt.

Beispiel 1

2-[(E,Z)-(1S,5S,6R,7R)-7-Hydroxy-6-(tert.-butyldiphenylsilyloxymethyl)-bicyclo[3.3.0] octan-3-yliden]-essigsäure-(+)-8-phenylmenthylester

306 mg (0,8 mmol) (+)-8-Phenylmenthylphosphonoacetat-P,P-dimethylester werden in 3 ml wasserfreiem Tetrahydrofuran unter Argon gelöst und auf + 5°C gekühlt. Nach Zugabe von 67 mg (0,6 mmol) frisch im Vakuum sublimierten Kalium-tert.-butylat wird 10 min. bei 5°C gerührt und nach Kühlung auf – 35°C eine Lösung von 204 mg (0,5 mmol) (1R,5S,6R,7R)-7-Hydroxy-6-(tert.-butyldiphenylsilyloxymethyl)-bicyclo[3.3.0]octan-3- on in 2 ml wasserfreiem Toluol langsam zugetropft. Nach 90 h Rühren bei – 30°C wird mit überschüssiger 0,5 N HCl versetzt und die resultierende Mischung in 100 ml Essigester aufgenommen. Die organische Phase wird nacheinander mit verdünnter HCl, ges. $NaHCO_3$-Lösung und Sole gewaschen. Nach Trocknen der organischen Phase und Abdampfen des Lösungsmittels bleibt ein helles Öl zurück, das anschließend an $SiO_2$ chromatographiert wird (Laufmittel Hexan/Essigester), wobei 313 mg = 94% homogenes Produkt erhalten wird. Da sich das E/Z-Verhältnis auf dieser Stufe nicht gaschromatographisch bestimmen läßt, wird der Ester zum Alkohol reduziert (siehe nächstes Beispiel).

Beispiel 2

2-[(E,Z)-(1S,5S,6R,7R)-7-Hydroxy-6-(tert.-butyldiphenylsilyloxymethyl)-bicyclo[3.3.0] octan-3-yliden]-ethan-1-ol

313 mg (0,471 mmol) des Esters aus Beispiel 1 werden in 6 ml wasserfreiem Toluol gelöst. Bei 0°C werden unter Argon 2,3 ml einer 1,25 M DIBAH-Lösung in Toluol zugetropft. Nach 1stündigem Rühren bei 0°C werden 0,5 ml Isopropanol und anschließend 0,5 ml $H_2O$ zugetropft. Nach 45 min. Rühren, Aufnehmen in Essigester und Trocknen der organischen Phase wird an $SiO_2$ (Laufmittel Hexan/Essigester) chromatographiert, wobei 120 mg = 58% der gewünschten Verbindung als Öl isoliert werden. Nach Silylierung mit MSTFA wird das E/Z-Verhältnis gaschromatographisch (25 m Quarzkap. CPSil 19CB) mit 86 : 10 (neben 4% Verunreinigungen) bestimmt.

Beispiel 3

2-[(E,Z)-(1S,5S,6R,7R)-7-Hydroxy-6-(tert.-butyldiphenylsilyloxymethyl)-bicyclo[3.3.0] octan-3-yliden]-essigsäure-(–)-8-phenylmenthylester

Molare Mengen, Ausführung und Aufarbeitung wie in Beispiel 1 beschrieben. Anstelle des (+)-8-Phenylmethylphosphonacetat-P,P-dimethylesters wird das Enantiomer, der (–)-8-Phenylmenthylphosphonoacetat-P,P-dimethylester verwandt. Chromatographie an $SiO_2$ (Laufmittel Hexan/Essigester) ergibt 322 mg = 97% der gewünschten Verbindung, die zur Bestimmung des E/Z-Verhältnisses reduziert wird (siehe nächstes Beispiel).

Beispiel 4

2-[(E,Z)-(1S,5S,6R,7R)-7-Hydroxy-6-(tert.-butyldiphenylsilyloxymethyl)-bicyclo[3.3.0] octan-3-yliden]ethan-1-ol

Ausführung der Reduktion unter Verwendung von 322 mg (0,484 mmol) des Esters aus Beispiel 3. Molare Verhältnisse und Bedingungen entsprechen denen im Beispiel 2. Nach Chromatographie an $SiO_2$ (Laufmittel Hexan/Essigester) bleiben 138 mg = 65% farbloses Öl zurück. Nach Silylieren der freien Hydroxylgruppen mit MSTFA wird das E/Z-Verhältnis gaschromatographisch mit 8 : 88 (neben 4% Verunreinigungen) bestimmt (25 m Quarzkap. CP-Sil 19 CB).

Beispiel 5

2-{(E,Z)-(1S,5S,6S,7R)-7-(tert.-Butyldimethylsilyloxy)-6-[(3S, 4S)-3-(tert.-butyldimethylsilyloxy) -4-methyl-nona-1,6-diinyl]-bicyclo[3.3.0] octan-3-yliden}-essigsäure-(+)-8-phenylmenthylester

306 mg (0.8 mmol) (+)-8-Phenylmenthylphosphonoacetat-P,P-dimethylester werden mit 258 mg (0,5 mmol) (1R,5S,6S,7R)-7-(tert.-Butyldimethylsilyloxy)-6-[(3S,4S)-3-(tert.-butyldimethylsilyloxy)-4-methyl-nona-1,6-diinyl]-bicyclo[3.3.0]octan-3-on wie in Beispiel 1 beschrieben umgesetzt. 87 h bei – 30°C rühren. Nach dem Aufarbeiten und Chromatographieren an $SiO_2$ (Laufmittel Hexan/Essigester) bleiben 370 mg = 95% der gewünschten Verbindung als Öl zurück.

Beispiel 6

2-{(E,Z)-(1S,5S,6S,7R)-7-(tert.-Butyldimethylsilyloxy)-6-[(3S, 4S)-3-(tert.-butyldimethylsilyloxy) -4-methyl-nona-1,6-diinyl]-bicyclo[3.3.0]octan-3-yliden}-ethan-1-ol

335 mg (0,433 mmol) des Esters aus Beispiel 5 werden wie Beispiel 2 beschrieben mit 1,25 ml einer 1,2 M Lösung DIBAH in Toluol reduziert. Aufarbeiten und Chromatographieren an $SiO_2$ (Laufmittel Hexan/Essigester) ergibt 199 mg = 84% farbloses Öl.

Nach Silylieren der primären Hydroxylgruppe mit MSTFA wird das E/Z-Verhältnis gaschromatographisch mit 80 : 20 bestimmt (25 m CP Sil 8 CB).

Beispiel 7

2-{(E,Z)-(1S,5S,6S,7R)-7-(tert.-Butyldimethylsilyloxy)-6-[(3S, 4S)-3-(tert.-butyldimethylsilyloxy) -4-methyl-nona-1,6-diinyl]-bicyclo-[3.3.0]octan-3-yliden}-essigsäure-(–)-8-phenylmenthylester

Mengen, Versuchsdurchführung und -dauer wie Beispiel 5 beschrieben. Anstelle des (+)-8-Phenylmenthylphosphonacetat-P,P-dimethylesters wird hier das (–)-Enantiomere verwendet. Nach Aufarbeiten und Chromatographieren an $SiO_2$ (Laufmittel Hexan/Essigester) verbleiben 354 mg = 91% der gewünschten Verbindung als Öl. Da die Substanz gaschromatographisch nicht zu untersuchen ist, wird der Ester reduziert (siehe Beispiel 8).

Beispiel 8

2-{(E,Z)-(1S,5S,6S,7R)-7-(tert.-Butyldimethylsilyloxy)-6-[(3S, 4S)-3-(tert.-butyldimethylsilyloxy) -4-methyl-nona-1,6-diinyl]-bicyclo-[3.3.0]octan-3-yliden}-ethan-1-ol

Versuchsdurchführung und Aufarbeitung wie Beispiel 6 beschrieben. Es werden 316 mg (0,408 mmol) des Esters aus Beispiel 7 und 1,25 ml einer 1,2 M DIBAH-Lösung in Toluol verwendet. Nach Chromatographie an $SiO_2$ (Laufmittel Hexan/Essigester) bleiben 168 mg = 75% zurück.
Gaschromatographie unter Bedingungen wie Beispiel 6 ergibt ein E/Z-Verhältnis von 20 : 78 (neben 2% Verunreinigung).

Beispiel 9

2-[(E,Z)-(1S,5S,6R,7R)-7-Hydroxy-6-(tert.-butyldimethylsilyloxymethyl)-bicyclo[3.3.0] octan-3-yliden]-essigsäure-(+)-8-phenylmenthylester

306 mg (0,8 mmol) (+)-8-Phenylmenthylphosphonoacetat-P,P-dimethylester werden wie im Beispiel 1 beschrieben mit 142 mg (0,5 mmol) (1R,5S,6R,7R)-7-Hydroxy-6-(tert.-butyldimethylsilyloxymethyl)-bicyclo[3.3.0]octan-3-on umgesetzt. 115 h bei – 30°C. Aufarbeiten und Chromatographieren (Laufmittel Hexan/Essigester) wie üblich. Zurück bleiben 190 mg = 70% der gewünschten Verbindung. Das E/Z-Verhältnis beträgt nach gaschromatographischer Bestimmung (25 m CP Sil 8 CB, Silylierung mit MSTFA) 91 : 4 (5% Verunreinigung).

Beispiel 10

2-[(E,Z)-(1S,5S,6R,7R)-7-Hydroxy-6-(tert.-butyldimethylsilyloxymethyl)-bicyclo[3.3.0] octan-3-yliden]-essigsäure-(–)-8-phenylmenthylester

306 mg (0,8 mmol) (-)-8-Phenylmenthylphosphonoacetat-P,P-dimethylester werden wie beschrieben mit 142 mg (0,5 mmol) (1R, 5S,6R,7R)-7-Hydroxy-6-(tert.-butyldimethylsilyloxymethyl)-bicyclo[3.3.0]octan-3-on umgesetzt. 115 h bei – 30°C. Wie üblich wird aufgearbeitet und chromatographiert (Laufmittel Hexan/Essigester). Es werden 194 mg = 72% einheitliches Produkt erhalten, dessen E/Z-Verhältnis nach Gaschromatographie (Bedingungen siehe Beispiel 9) 4 : 94 beträgt (2% Verunreinigungen).

Beispiel 11

2-[(E,Z)-(1S,5S,6R,7R)-7-(tert.-Butyldimethylsilyloxy)-6-(tert.-butyldimethylsilyloxymethyl)-bicyclo[3.3.0]octan-3-yliden]-essigsäure-(+)-8-phenylmenthylester

306 mg (0,8 mmol) (+)-8-Phenylmenthylphosphonoacetat-P,P-dimethylester werden mit 199 mg (0,5 mmol) (1R,5S,6R,7R)-7-(tert.-butyldimethylsilyloxy)-6-(tert.-butyldimethylsilyloxymethyl)-bicyclo[3.3.0]octan-3-on unter den Bedingungen wie im Beispiel 1 beschrieben umgesetzt. 94 h Rühren bei – 25°C. Nach Aufarbeiten und Chromatographieren wie beschrieben bleiben 310 mg = 94% der gewünschten Verbindung zurück. Laut Gaschromatographie (25 m CP Sil 8 CB) beträgt das E/Z-Verhältnis 78 : 22.

Beispiel 12

2[-(E,Z)-(1S,5S,6R,7R)-7-(tert.-Butyldimethylsilyloxy)-6-(tert.-butyldimethylsilyloxymethyl)-bicyclo[3.3.0]octan-3-yliden]-essigsäure-(–)-8-phenylmenthylester

306 mg (0,8 mmol) (–)-8-Phenylmenthylphosphonoacetat-P,P-dimethylester werden mit 199 mg (0,5 mmol) (1R,5S,6R,7R)-7-tert.-Butyldimethylsilyloxy)-6-(tert.-butyldimethylsilyloxymethyl)-bicyclo[3.3.0]octan-3-on wie beschrieben umgesetzt. 115 h Rühren bei – 30°C. Nach Aufarbeiten und Chromatographie werden 299 mg = 91% der gewünschten Substanz erhalten. Die Gaschromatographie (25 m CP Sil 8 CB) zeigt ein E/Z-Verhältnis von 15 : 85 an.

Beispiel 13

2-[(E/Z)-(1S,5S,6R,7R)-7-(tert.-Butyldimethylsilyloxy)-6-(tert.-butyldimethylsilyloxymethyl)-bicyclo[3.3.0]octan-3-yliden]-essigsäure-(–)-trans-2-phenyl-cyclohexylester

261 mg (0,8 mmol) (–)-trans-2-Phenyl-cyclohexylphosphonacetat-P,P-dimethylester werden wie im Beispiel 1 beschrieben mit 199 mg (0,5 mmol) (1R,5S,6R,7R)-7-(tert.-Butyldimethylsilyloxy)-6-(tert.-butyldimethylsilyloxymethyl)- bicyclo[3.3.0]octan-3-on umgesetzt. Nach 87 stündigem Rühren bei – 30°C, Aufarbeiten und Chromatographieren bleiben 280 mg = 93% gewünschte Verbindung als helles Öl zurück. Die gaschromatographische Auftrennung ergibt ein E/Z-Verhältnis von 77 : 23 (25 m CP Sil 8 CB).

## Patentansprüche

1. Verfahren zur Herstellung von E/Z-Gemischen von 2-(Bicyclo[3.3.0] octan-3-yliden)-essigsäurederivaten der Formel I,

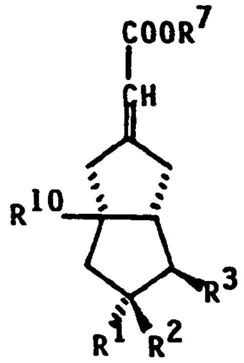

(I),

in denen entweder der E- oder der Z-Anteil überwiegt, worin

R¹ — $OCH_3$, $OC_2H_5$,

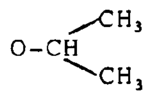

und, wenn $R^2$ Wasserstoff darstellt, den Rest $OR^4$ mit $R^4$ in der Bedeutung eines Wasserstoffs, Trialkylsilyls, Diphenyl-alkylsilyls, tert.-Butyl-methoxy-phenylsilyls, Trityls, Tetrahydropyranyls, $C_7$-$C_{11}$-Aroyls oder $C_1$-$C_6$-Acyls,

$R^2$ — $OCH_3$, $OC_2H_5$,

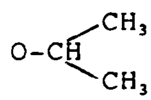

,

$R^1$ und $R^2$ — gemeinsam die Reste

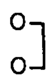 , 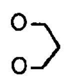 , 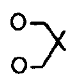 oder 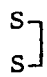 ,

$R^3$ — Wasserstoff, $—CH_2OR^4$ mit $R^4$ in der oben angegebenen Bedeutung oder $—A\text{-}W\text{-}D\text{-}E\text{-}R^5$, worin

A — eine trans-CH=CH—, eine —CH=CBr— oder —C≡C-Gruppe,

W — die Reste

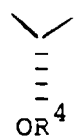 oder 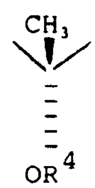

mit

$R^4$ — in der oben angegebenen Bedeutung,

D — eine geradkettige Alkylengruppe mit 1-7 C-Atomen oder eine verzweigkettige Alkylengruppe mit 2-7 C-Atomen,

E — eine —C≡C—, $—CH{=}CR^6$, $—O\text{-}R^5$ oder $—S\text{-}R^5$-Gruppe,

$R^5$ — eine Alkylgruppe mit 1-6 C-Atomen,

$D\text{-}E\text{-}R^5$ — eine Cycloalkylgruppe mit 3-8 C-Atomen oder den Rest

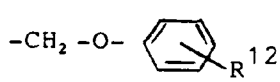

,

$R^6$ — eine Alkylgruppe mit 1-6 C-Atomen oder Halogen,

$R^{12}$ — Wasserstoff, 4-Halogen oder 3-Trifluormethyl sein können,

| | |
|---|---|
| $R^7$ | (+)- oder (–)-Menthyl, (+)- oder (–)-8-Arylmenthyl, (+)-8-Arylneomenthyl, (+)- oder (–)-trans-2-Arylcycloalkyl mit 3-8 C-Atomen im cycloalkyl und mit Aryl als gegebenenfalls substituiertes Phenyl, 1-oder 2-Naphthyl oder 1-, 2- oder 9-Anthranyl, gegebenenfalls substituiertes Bornyl oder 3-Methoxy-1, 3,5-estratrien-17β-yl und |
| $R^{10}$ | Wasserstoff, Methyl, Ethinyl, 1-Propinyl oder den Rest $—C≡C-(CH_2)_m-R^{11}$, worin |
| m | 2-20 und |
| $R^{11}$ | Wasserstoff, Azido, Amino, Methylamino, Benzylamino, Carboxyl, Methoxycarbonyl, Ethoxycarbonyl, Benzyloxycarbonyl, Hydroxy, Cyano, Brom, Chlor oder Jod darstellen, |

bedeuten, dadurch gekennzeichnet, daß man ein Bicyclo-[3.3.0]octan-3-on der Formel II,

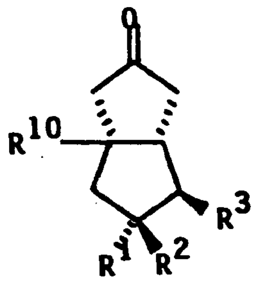

(II),

worin $R^1$, $R^2$, $R^3$ und $R^{10}$ die oben angegebenen Bedeutungen haben, mit einem chiralen Phosphonat der Formel III,

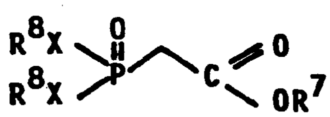

(III),

worin

| | |
|---|---|
| X | Sauerstoff oder den Rest $—N(R^9)—$, |
| $R^8$ | einen geradkettigen oder verzweigten Alkylrest mit 1-6 C-Atomen, Phenyl oder 2,2,2-Trifluorethyl oder beide Reste $R^8$ gemeinsam 1,2-Cyclohexyliden und |
| $R^9$ | Wasserstoff, Methyl, Ethyl oder Benzyl bedeuten und |
| $R^7$ | die oben angegebene Bedeutung hat, |

in Gegenwart eines Deprotonierungsmittels umsetzt.

## Claims

1. Process for the preparation of E/Z mixtures of 2-(bicyclo[3.3.0]octan-3-ylidene)acetic acid derivatives of the formula I

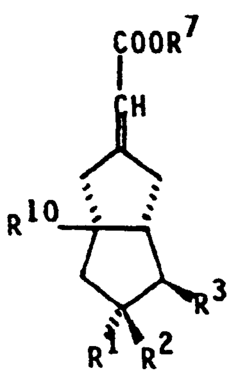

(I)

in which either the E or the Z portion predominates, wherein

$R^1$ represents $OCH_3$, $OC_2H_5$ or

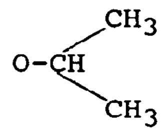

and, when $R^2$ is hydrogen, $R^1$ represents the radical $OR^4$ in which $R^4$ represents hydrogen, trialkylsilyl, diphenylalkylsilyl, tert-butylmethoxyphenylsilyl, trityl, tetrahydropyranyl, $C_7$-$C_{11}$-aroyl or $C_1$-$C_6$-acyl,

$R^2$ represents $OCH_3$, $OC_2H_5$ or

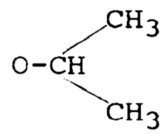 ,

$R^1$ and $R^2$ together represent the radicals

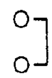 , 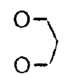 , 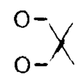 or 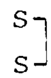 ,

$R^3$ represents hydrogen, $—CH_2OR^4$ in which $R^4$ is as defined above, or $—A-W-D-E-R^5$, wherein

A may be a <u>trans</u>-CH=CH—, a —CH=CBr— or a —C≡C group,

W may be the radical

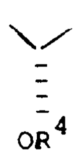 or 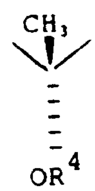

in which

$R^4$ is as defined above,

D may be a straight-chain alkylene group having from 1 to 7 carbon atoms or a branched-chain alkylene group having from 2 to 7 carbon atoms,

E may be a —C≡C—, $—CH=CR^6—$, $—O-R^5$ or $—S-R^5$ group,

$R^5$ may be an alkyl group having from 1 to 6 carbon atoms,

$D-E-R^5$ may be a cycloalkyl group having from 3 to 8 carbon atoms or the radical

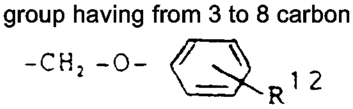 ,

$R^6$ may be an alkyl group having from 1 to 6 carbon atoms or halogen,

$R^{12}$ may be hydrogen, 4-halogen or 3-trifluoromethyl,

$R^7$ represents (+)- or (−)-menthyl, (+)- or (−)-8-arylmenthyl, (+)-8-arylneomenthyl, (+)- or (−)-trans-2-arylcycloalkyl having from 3 to 8 carbon atoms in the cycloalkyl radical and with aryl as optionally substituted phenyl, 1- or 2-naphthyl or 1-, 2- or 9-anthranyl, or represents optionally substituted bornyl or 3-methoxy-1,3,5-oestratrien-17β-yl and

$R^{10}$ represents hydrogen, methyl, ethynyl, 1-propynyl or the radical $—C≡C-(CH_2)_m-R^{11}$, wherein

<u>m</u> is from 2 to 20 and

$R^{11}$ is hydrogen, azido, amino, methylamino, benzylamino, carboxy, methoxycarbonyl, ethoxycarbonyl, benzyloxycarbonyl, hydroxy, cyano, bromine, chlorine or iodine, characterised in that a bicyclo[3.3.0]octan-3-one of the formula II,

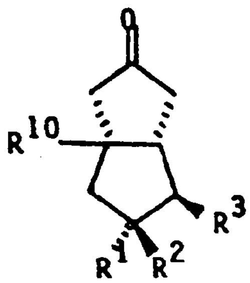

(II)

wherein $R^1$, $R^2$, $R^3$ and $R^{10}$ are as defined above, is reacted with a chiral phosphonate of the formula III,

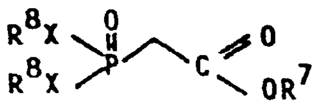

(III)

wherein

X represents oxygen or the radical —N($R^9$)—,
$R^8$ represents a straight-chain or branched alkyl radical having from 1 to 6 carbon atoms, phenyl or 2,2,2-trifluoroethyl or the two radicals $R^8$ together represent 1,2-cyclohexylidene and
$R^9$ represents hydrogen, methyl, ethyl or benzyl and
$R^7$ is as defined above,
in the presence of a deprotonating agent.

## Revendications

1. Procédé pour préparer des mélanges E/Z à proportion prédominante soit de l'isomère E soit de l'isomère Z de dérivés de l'acide (bicyclo[3.3.0]octylidène-3)-2 acétique répondant à la formule I :

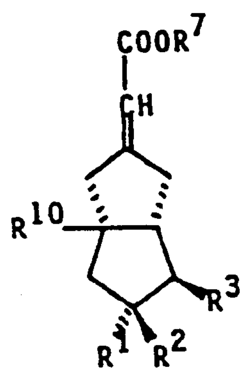

(I)

dans laquelle

$R^1$ représente $OCH_3$, $OC_2H_5$,

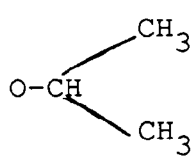

ou, lorsque $R^2$ est l'hydrogène, un radical $OR^4$ dont le symbole $R^4$ désigne l'hydrogène, un trialkyl-silyle, un diphényl-alkylsilyle, un tert-butyl-méthoxy-phényl-silyle, un trityle, un tétrahydropyrannyle, un aroyle en $C_7$-$C_{11}$ ou un acyle en $C_1$-$C_6$,

$R^2$ représente $OCH_3$, $OC_2H_5$ ou

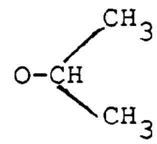 ,

$R^1$ et $R^2$ forment ensemble un radical

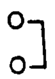 , 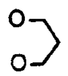 , 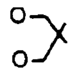 ou 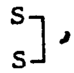 ,

$R^3$ représente l'hydrogène, un radical $—CH_2OR^4$ dont le symbole $R^4$ a la signification qui lui a été donnée ci-dessus, ou un radical $—A-W-D-E-R^5$ dans lequel :

A représente un radical —CH=CH— trans, un radical —CH=CBr— ou un radical —C≡C—,

W représente un radical

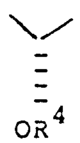 ou un radical 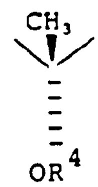

dans lesquels

$R^4$ a la signification qui lui a été donnée ci-dessus,

D représente un alkylène linéaire contenant de 1 à 7 atomes de carbone ou un alkylène ramifié contenant de 2 à 7 atomes de carbone,

E représente un radical —C≡C—, $—CH=CR^6—$, $—O-R^6—$ ou $—S-R^6—$,

$R^5$ représente un alkyle contenant de 1 à 6 atomes de carbone,

$D-E-R^5$ représente un radical cycloalkyle contenant de 3 à 8 atomes de carbone ou un radical

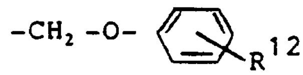 .

$R^6$ représente un alkyle contenant de 1 à 6 atomes de carbone ou un halogène, et

$R^{12}$ représente l'hydrogène, un halogène en 4 ou un trifluorométhyle en 3,

$R^7$ représente un menthyle (+) ou (–), un aryl-8 menthyle (+) ou (–), un aryl-8 néomenthyle (+), un aryl-2 cycloalkyle trans (+) ou (–) dont le cycloalkyle contient de 3 à 8 atomes de carbone et dont l'aryle est un phényle éventuellement substitué, un naphtyle-1 ou -2 ou un anthryle-1, -2 ou -9, un bornyle éventuellement substitué ou un méthoxy-3 estratriène-1,3,5 yle-17β, et

$R^{10}$ représente l'hydrogène, un radical méthyle, éthynyle ou propyne-1 yle ou un radical $—C≡C-(CH_2)m-R^{11}$ dans lequel

m désigne un nombre de 2 à 20 et

$R^{11}$ représente l'hydrogène, un radical azido, amino, méthylamino, benzylamino, carboxy, méthoxycarbonyle, éthoxycarbonyle, benzyloxycarbonyle, hydroxy ou cyano, le brome, le chlore ou l'iode,

procédé caractérisé en ce qu'on fait réagir, en présence d'un agent de déprotonation, une bicyclo[3.3.0]octanone-3 répondant à la formule II :

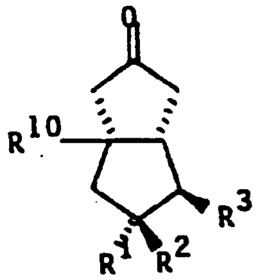

(II),

dans laquelle $R^1$, $R^2$, $R^3$ et $R^{10}$ ont les significations qui leur ont été données ci-dessus, avec un phosphonate chiral répondant à la formule III :

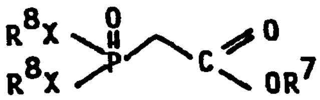

(III),

dans laquelle

X représente l'oxygène ou un radical —N($R^9$)—,

$R^8$ représente un alkyle linéaire ou ramifié qui contient de 1 à 6 atomes de carbone, un phényle ou un trifluoro-2,2,2 éthyle, ou les deux symboles $R^8$ forment ensemble un radical cyclohexylène-1,2,

$R^9$ représente l'hydrogène ou un radical méthyle, éthyle ou benzyle et

$R^7$ a la signification qui lui a été donnée ci-dessus.